(19) Europäisches Patentamt
European Patent Office
Office européen des brevets

(11) Veröffentlichungsnummer: **0 325 266 B1**

(12) **EUROPÄISCHE PATENTSCHRIFT**

(45) Veröffentlichungstag der Patentschrift: **11.08.93**

(51) Int. Cl.$^5$: **A61K 6/08**

(21) Anmeldenummer: **89100951.6**

(22) Anmeldetag: **20.01.89**

# (54) Dentalmassen.

(30) Priorität: **20.01.88 DE 3801511**

(43) Veröffentlichungstag der Anmeldung:
**26.07.89 Patentblatt 89/30**

(45) Bekanntmachung des Hinweises auf die Patenterteilung:
**11.08.93 Patentblatt 93/32**

(84) Benannte Vertragsstaaten:
**AT BE CH DE ES FR GB IT LI NL SE**

(56) Entgegenhaltungen:
**EP-A- 0 059 649**
**EP-A- 0 173 567**
**EP-A- 0 184 095**
**GB-A- 2 180 247**

(73) Patentinhaber: **THERA Patent GmbH & Co. KG**
**Gesellschaft für industrielle Schutzrechte**
**Am Griesberg 2**
**W-8031 Seefeld 1(DE)**

(72) Erfinder: **Schmitt, Werner, Dr.**
**Prinzenweg 10**
**W-8130 Starnberg(DE)**
Erfinder: **Jochum, Peter, Dr.**
**Pointweg 5**
**W-8031 Seefeld 2(DE)**
Erfinder: **Ellrich, Klaus, Dr.**
**Auingerstrasse 16**
**W-8031 Wörthsee(DE)**

(74) Vertreter: **Abitz, Walter, Dr.-Ing. et al**
**Abitz, Morf, Gritschneder, Freiherr von Wittgenstein Postfach 86 01 09**
**W-8000 München 86 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3. 10/3.6/3.3. 1)

## Beschreibung

Die Erfindung betrifft Dentalmassen, insbesondere Zahnfüllmassen, die in zwei Schritten härtbar sind.

Fast alle handelsüblichen kosmetisch anspruchsvollen Dentalmassen, insbesondere die Zahnfüllmassen, bestehen aus Kunststoffen, die mit organischen oder anorganischen Füllkörpern versehen sind (sogenannte Composites). Der Füllkörperanteil kann hierbei von 30 bis 90 Gew.-% variieren. Insbesondere auf dem Gebiet der sogenannten Seitenzahn-Composites hat es sich gezeigt, dass es wünschenswert ist, Zahnfüllmassen so hoch wie möglich mit harten anorganischen Füllstoffen zu füllen, um der fertigen Restauration eine ausreichende Härte zu verleihen. Besonders vorteilhaft hat sich hierbei die Verwendung von silanisierten feinkörnigen Füllstoffen wie Quarz oder röntgenopaken Gläsern zur Herstellung abrasionsbeständiger Seitenzahncomposites gezeigt. Solche Zahnfüllmassen enthalten vorteilhaft 80 bis 90 Gew.-% anorganisches Füllmaterial. Die verwendeten Füllstoffe haben üblicherweise eine mittlere Korngrösse von 1 bis 15 µm. In der Regel werden auch wesentlich feinere Füllstoffe im Bereich von 0,02 bis 0,05 µm gleichzeitig mit den obigen Füllstoffen eingesetzt, um die Massen ausreichend plastisch zu machen.

Besonders vorteilhaft hat sich erwiesen, sogenannte Hybrid-Composites im Seitenzahncomposite-Gebiet einzusetzen, da bei gleichzeitigem Einsatz von 5 bis 25 Gew.-% Füllstoffen mit einer mittleren Korngrösse von 0,02 bis 0,05 µm und 65 bis 85 Gew.-% Füllkörper mit einer mittleren Korngrösse von 1 bis 15 µm besonders abrasionsresistente Zahnfüllmassen erhalten werden. Hier hat sich einerseits gezeigt, dass solche Seitenzahncomposites über eine ausreichende Härte verfügen, um den Belastungen des Materials im Mundmilieu ausreichend lange standzuhalten, zum anderen hat sich aber das Problem ergeben, dass nach Legen der Füllung und Aushärten, üblicherweise mit sichtbarem Licht, bestehende Überschüsse nur noch schlecht vom umgebenden Zahnmaterial unterschieden werden können. Dies beruht zum einen auf der möglichen zahnähnlichen Einfärbung des Materials (im Unterschied zur klassischen Amalgamfüllung), zum anderen aber auch auf der zahnähnlichen Härte solch harter Zahnfüllmassen.

Die gleichen Probleme wie beim Legen von Zahnfüllungen hat der behandelnde Zahnarzt aber auch beim Einzementieren von Inlays, Onlays sowie Verblendschalen aus hinreichend transparentem Material, z.B. Porzellan oder Kunststoff. Hierzu werden bisher vorzugsweise lichthärtende Materialien eingesetzt, die z.T. über einen nachgeschalteten Redox-Prozess noch zu einer Nachhärtung führen. Da die erste Fixierung aber immer mit einer Photopolymerisation eingeleitet wird, bestehen hierbei wie beim Legen von Zahnfüllungen die Probleme beim Ausarbeiten der Füllung.

So ist insbesondere das Entfernen von Überschüssen sowie das Einschleifen der Okklusion für den behandelnden Zahnarzt sehr schwierig geworden und nur unter grossem Zeitaufwand und grossem Materialverschleiss des Polierinstruments durchzuführen. Für diesen Zweck können nur sehr teure Diamant-Instrumente eingesetzt werden, und durch den Einsatz dieser Instrumente besteht bei der gelegten Füllung immer die Gefahr, dass beim Polieren der Schmelzrand, also der Randbereich des noch bestehenden Zahnes, gefährdet wird.

Aus den beiden britischen Patentanmeldungen 21 80 245 und 21 80 246 sind lichthärtende Zahnfüllmassen bekannt, die neben einer Verbindung mit wenigstens einer ethylenisch ungesättigten Doppelbindung wenigstens einen Photopolymerisationsinitiator auf Ketalbasis, wenigstens einen Photopolymerisationsinitiator auf Thioxanthonbasis und ein Reduktionsmittel enthalten. Der Photopolymerisationsinitiator auf Ketalbasis dient für UV-Licht unterhalb 400 nm, während der Photopolymerisationsinitiator auf Thioxanthonbasis für sichtbares Licht einer Wellenlänge von mehr als 400 nm dient. Diese Zusammensetzungen haben den Vorteil, daß sie sowohl durch sichtbares als auch durch UV-Licht gehärtet werden können.

Aufgabe der Erfindung ist daher die Bereitstellung von neuen Dentalmassen, besonders Zahnfüllmassen, die in zwei Schritten härtbar sind und die nicht die Nachteile des Standes der Technik aufweisen. Insbesondere sollen die neuen Dentalmassen leicht verarbeitbar sein und dennoch zu einer ausgezeichneten Endhärte des Dentalmaterials führen.

Gegenstand der Erfindung sind somit Dentalmassen, enthaltend

(a) 5 bis 70 Gew.-%, bezogen auf (a) + (b), ethylenisch ungesättigtes, polymerisierbares Monomeres und/oder Polymeres,

(b) 30 bis 95 Gew.-%, bezogen auf (a) + (b), Füllstoffe,

(c) gegebenenfalls Pigmente, röntgenopake Zusatzstoffe und/oder Thixotropie-Hilfsmittel,

(d) 0,05 bis 3 Gew.-%, bezogen auf (a), einer Photoinitiatorkomponente I mit einem Lichtabsorptionsmaximum < 450 nm und einer molaren Extinktion der Lichtabsorption von < 10 bei Wellenlängen von 470 nm und darüber sowie

(e) 0,05 bis 3 Gew.-%, bezogen auf (a), einer Photoinitiatorkomponente II mit einer molaren Extinktion der Lichtabsorption von > 20 bei mindestens einer Wellenlänge von > 450 nm,

dadurch gekennzeichnet, daß sie die Photoinitiatorkomponente II nach Art und Menge so ausgewählt

enthalten, daß die Photoinitiatorkomponente II zu einem ausgehärteten Material führt, das maximal 70%, vorzugsweise maximal 50 % derjenigen Härte aufweist, die das gleiche Material nach Aushärtung nur mit der jeweils verwendeten Photoinitiatorkomponente I aufweist,
sowie ein Verfahren zur Herstellung dieser Dentalmassen durch Vermischen der Bestandteile (a), (b), (d), (e) und gegebenenfalls (c).

Die Erfindung betrifft weiter ein Verfahren zum Härten dieser Dentalmassen, wobei man die Massen in einem ersten Aushärtungsschritt mit Licht aus dem Wellenlängenbereich von im wesentlichen > 450 nm, vorzugsweise > 470 nm, und, nach Oberflächengestaltung der vorgehärteten Dentalmasse, in einem zweiten Aushärtungsschritt mit Licht, das deutliche Anteile von Wellenlängen < 450 nm aufweist, bestrahlt.

Gegenstand der Erfindung ist ausserdem die Verwendung einer Kombination aus einer Photoinitiatorkomponente I mit einem Lichtabsorptionsmaximum < 450 nm und einer molaren Extinktion der Lichtabsorption von < 10 bei Wellenlängen von 470 nm und darüber und aus einer Photoinitiatorkomponente II mit einer molaren Extinktion der Lichtabsorption von > 20 bei mindestens einer Wellenlänge von > 450 nm zur Herstellung von in zwei Schritten härtbaren Dentalmassen.

Die erfindungsgemäss verwendeten Photoinitiatorkomponenten I und II sind vorzugsweise jeweils in einer Menge von 0,1 bis 2 Gew.-%, ganz besonders von 0,5 bis 1,5 Gew.-%, bezogen auf die Menge an (a), enthalten.

Die erfindungsgemässen Photoinitiatorkomponenten I und II müssen nicht in gleicher Konzentration eingesetzt werden, vielmehr hängt die optimale Konzentration jeder Komponente ab von der Lichtintensität der zur Aushärtung benutzten Lampe sowie vom ausgewählten Wellenlängenbereich für die Aktivierung der Photoinitiatorkomponente II. Unter Endhärte wird hierbei definitionsgemäss die Oberflächenhärte verstanden, die bei vollständiger Aushärtung maximal erhalten werden kann. Die Messung der Oberflächenhärte kann beispielsweise nach DIN-Spezifikation 53456 erfolgen.

Als Photoinitiatorkomponente I eignen sich alle bekannten Photoinitiatoren, die ein Lichtabsorptionsmaximum < 450 nm und eine molare Extinktion der Lichtabsorption von < 10 bei Wellenlängen von 470 nm und darüber besitzen. Besonders bevorzugt sind Photoinitiatoren, die ein Absorptionsmaximum < 430 nm, eine molare Extinktion von < 10 bei Wellenlängen von 470 nm und darüber und eine molare Extinktion bei 400 nm > 25 haben. Als geeignet haben sich erwiesen Monoacylphosphinoxide und Monoacylphosphinsulfide, wie sie in den europäischen Patentveröffentlichungsschriften 73413, 7508, 47902 und 57474 beschrieben sind, z.B. 2,4,6-Trimethylbenzoyldiphenylphosphinoxid. Besonders geeignet sind vor allem Bisacylphosphinoxide, wie sie aus der europäischen Patentveröffentlichungsschrift 184095 bekannt sind. Diese Bisacylphosphinoxide haben die allgemeine Formel

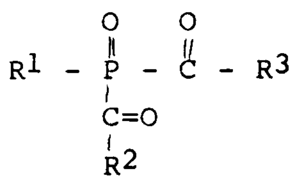

worin bedeuten:

$R^1$ einen gradkettigen oder verzweigten $C_{1-18}$Alkylrest,
einen Cyclohexyl-, Cyclopentyl-, Phenyl-, Naphthyl- oder Biphenylylrest,
einen Cyclopentyl-, Cyclohexyl-, Phenyl-, Naphthyl- oder Biphenylylrest, der substituiert ist durch F, Cl, Br, J, $C_1$-$C_{12}$-Alkyl und/oder $C_1$-$C_{12}$-Alkoxyl, oder
einen S- oder N-haltigen 5- oder 6-gliedrigen heterocyclischen Ring und
$R^2$ und $R^3$, die gleich oder verschieden sind,
einen Cyclohexyl-, Cyclopentyl-, Phenyl-, Naphthyl- oder Biphenylylrest,
einen Cyclopentyl-, Cyclohexyl-, Phenyl-, Naphthyl- oder Biphenylylrest, der substituiert ist durch F, Cl, Br, J, $C_1$-$C_4$-Alkyl und/oder $C_1$-$C_4$-Alkoxyl, oder
einen S- oder N-haltigen, 5- oder 6-gliedrigen, heterocyclischen Ring; oder
$R^2$ und $R^3$ miteinander zu einem Ring verknüpft sind, der 4 bis 10 Kohlenstoffatome enthält und durch 1 bis 6 $C_{1-4}$-Alkylreste substituiert sein kann.

Dabei sind besonders solche Verbindungen bevorzugt, bei denen in der allgemeinen Formel der Bisacylphosphinoxide $R^1$ Decyl, Phenyl, Naphthyl, 4-Biphenylyl, 2-Methylphenyl, 1-Methylnaphthyl, 2,5-Dimethylphenyl, 4-Propylphenyl, 4-Octylphenyl, 4-Chlorphenyl oder 4-Ethoxyphenyl und $R^2$ und $R^3$ Phenyl,

Naphthyl, 2,6-Dichlorphenyl, 2,6-Dimethoxyphenyl, 2-Methylnaphthyl, 2-Methoxynaphthyl, 2,6-Dimethylphenyl oder 2,4,6-Trimethylphenyl bedeuten.

Vorzugsweise sollen $R^2$ und $R^3$ die gleiche Bedeutung aufweisen. Beispiele für derartige Verbindungen sind Bis(2,6-dichlorbenzoyl)-phenylphosphinoxid, Bis(2,6-dichlorbenzoyl)-2,5-dimethylphenylphosphinoxid und Bis(2,6-dichlorbenzoyl)-4-n-propylphenylphosphinoxid.

Diese Photoinitiatoren haben ihr Absorptionsmaximum bei 360 bis 410 nm, d.h. sie weisen ≧ 400 nm noch beträchtliche molare Extinktionen auf (z.B. von 400 bis 3000 bei 400 nm); bei 470 nm und darüber besitzen diese Verbindungen keine messbare molare Extinktion mehr.

Als Photoinitiatorkomponente II eignen sich alle Photoinitiatoren des Stands der Technik mit einer molaren Extinktion der Lichtabsorption von > 20 bei mindestens einer Wellenlänge von > 450 nm, z.B. α-Diketone. Der Photoinitiator II hat vorzugsweise eine molare Extinktion von > 20 bei mindestens einer Wellenlänge zwischen > 450 und 500, besonders zwischen 470 und 500 nm. Insbesondere geeignet ist Campherchinon mit einem Absorptionsmaximum bei etwa 470 nm. Der Photoinitiator II sol vorzusweise nach Art und Menge so gewählt werden, dass er zu einem ausgehärteten Material führt, das maximal 70 %, vorzugsweise maximal 50 % der Härte aufweist, die das gleiche Material nach Aushärtung nur mit dem jeweils verwendeten Photoinitiator I aufweist. Der Photoinitiator II kann in diesem Falle auch zusammen mit einem Aktivator, z.B. einem tertiären Amin, verwendet werden, wenn dadurch die Härte des mit dem Photoinitiator II ausgehärteten Materials nicht mehr als 70 % des nur mit dem Photoinitiator I ausgehärteten gleichen Materials beträgt.

Besonders geeignet sind Dentalmassen, in welchen der Photoinitiator II nach Art und Menge so bemessen ist, dass bei Aushärtung mit Licht einer Wellenlänge im wesentlichen > 450 nm, bevorzugt im wesentlichen > 470 nm, eine Oberflächenhärte < 200 MPa, bevorzugt < 150 MPa, besonders bevorzugt < 100 MPa, erhalten wird.

Eine besonders geeignete Kombination von Photoinitiatoren besteht aus einer Mischung aus 0,05 bis 3 Gew.-% Bisacylphosphinoxid und 0,05 bis 3 Gew.-% Campherchinon, bezogen auf (a).

Es hat sich gezeigt, dass erfindungsgemässe Dentalmassen in zwei Schritten gehärtet werden können. In einem ersten Aushärtungsschritt wird durch Wahl der Lichtquelle oder durch Herausfiltern des Wellenlängenbereichs < 450 nm nur der Photoinitiator II angeregt. Durch das langwelligere, energieärmere Licht entstehen hierbei relativ wenig reaktive Radikale, die in Abwesenheit von Aktivatoren, wie Aminen, zu nur teilweiser Aushärtung der eingesetzten ethylenisch ungesättigten, polymerisierbaren Monomeren führen. Erfindungsgemäss beträgt die Härte des mit Photoinitiator II ausgehärteten Materials maximal 70 %, vorzugsweise maximal 50 % der Endhärte, die mit dem jeweils verwendeten Photoinitiator I allein bei Einsatz von niederwelligerem Licht ( < 450 nm) erhalten wird.

In einem zweiten Schritt wird durch Einsatz einer Lichtquelle mit deutlichen Anteilen an Licht < 450 nm die Endhärte des Materials erreicht. Dies kann beispielsweise durch Weglassen des im ersten Aushärtungsschritt eingesetzten optischen Kantenfilters und durch nochmalige Belichtung erfolgen.

Die Trennung der Wellenlängenbereiche für die zwei Aushärteschritte kann vorteilhaft auch mit Kantenfiltern im Bereich 450 bis 480 nm erfolgen. Die Wahl des Kantenfilters ist aber abhängig von der verwendeten Photoinitiatorkomponente II. Bei Einsatz von Campherchinon haben sich Kantenfilter im Bereich 465 bis 480 nm bewährt, insbesondere im Bereich 470 bis 480 nm.

Der Vorteil für den Benutzer solcher Dentalmassen liegt darin, dass z.B. der Zahnarzt beim Legen einer Füllung in einem ersten Aushärtungsschritt mit einer Lichtquelle mit Lichtanteilen im wesentlichen > 470 nm das Material fixieren und bis maximal 70 %, vorzugsweise maximal 50% der Endhärte anhärten kann. Insbesondere bei Seitenzahncomposites erhält man somit Härten, die noch mit Schnitzinstrumenten bearbeitet werden können; auch das anschliessende Polieren und Finieren kann mit deutlich geringeren Materialverlusten (am Instrument) durchgeführt werden. Nach Beendigung dieser Arbeiten kann der Benutzer die Dentalmassen durch Aushärten mit Licht von Wellenlängen < 450 nm (oder mit deutlichen Anteilen in diesem Bereich) dann auf die Endhärte bringen und hat somit eine ausgehärtete Füllung hergestellt, die den im Mundmilieu auftretenden Abrasionskräften ausreichend Widerstand bieten kann.

Ein weiteres vorteilhaftes Anwendungsgebiet für die erfindungsgemässen Massen sind lichthärtende Zementierungsmaterialien, mit denen man in einem ersten Aushärteschritt z.B. ausreichend transparente Inlays und Verblendschalen befestigen kann und anschliessend den Präparationsrand mit Schnitzinstrumenten unter deutlicher Materialschonung am Instrument und am Zahn bearbeiten kann. Erst nach Ausarbeitung kann dann mit einem zweiten Belichtungsschritt die Endhärte des Zementierungsmaterials erreicht werden.

In einer vorteilhaften Ausführungsform haben beide Photoinitiatoren I und II im Bereich 400 bis 500 nm einen ausreichenden Absorptionsbereich, so dass sie beide mit der gleichen handelsüblichen dentalen Bestrahlungseinheit aktiviert werden können. Die Trennung der beiden Aushärtungsschritte kann dann durch einfaches Auf- und Abstecken der Kantenfilter erfolgen.

Unter ethylenisch ungesättigten polymersierbaren Monomeren und Polymeren, die für Dentalzwecke geeignet sind, versteht man beispielsweise monomere und polymere Acrylate und Methacrylate. Bei polymerisierbaren Dentalmassen verwendet man insbesondere oft die langkettigen Monomeren der USA-Patentschrift 3066112 auf der Basis von Bisphenol-A und Glycidylmethacrylat oder deren durch Addition von Isocyanaten entstandene Derivate; geeignet sind auch die Acrylsäure- und Methacrylsäureester ein- oder mehrwertiger Alkohole, beispielsweise Methyl- und Ethylmethacrylat, insbesondere die Ester mehrwertiger Alkohole, wie Triethylenglykol-di(meth)acrylat, Ethylenglykol-di(meth)acrylat, Hexandiol-di(meth)acrylat und Trimethylolpropan-tri(meth)acrylat. Geeignet sind auch Verbindungen des Typs Bisphenol A - diethyl-(meth)acrylat und Bisphenol A - dipropyl(meth)acrylat.

Besonders geeignet sind die in der deutschen Patentschrift 2816823 genannten Diacryl- und Dimethacrylsäureester des Bis-hydroxymethyltricyclo[5.2.1.0.2,6]-decans. Verwendet werden können auch die Reaktionsprodukte aus Diisocyanaten und Hydroxyalkyl(meth)acrylaten, wie sie in der deutschen Offenlegungsschrift 2312559 beschrieben sind.

Selbstverständlich können auch Gemische aus Monomeren und/oder aus hieraus hergestellten ungesättigten Polymeren verwendet werden.

Dem Fachmann geläufige übliche Bestandteile der Dentalmassen sind, neben gesättigten und ungesättigten Polymeren, Pigmente, Farbstoffe und anorganische Füllstoffe. Anorganische Füllstoffe können beispielsweise Quarz, gemahlene Gläser, Kieselgele sowie Kieselsäuren oder deren Granulate sein. Sie können in einer Konzentration von 30 bis 95 Gew.-%, bezogen auf (a) + (b), enthalten sein. Ein bevorzugter Konzentrationsbereich der anorganischen Füllstoffe beträgt 60 bis 95 Gew.-%, insbesondere bevorzugt sind 75 bis 90 Gew.-%, bezogen auf (a) + (b).

In einer bevorzugten Ausführungsform besteht der Füllkörperanteil zum einen aus 1 bis 25 Gew.-%, bevorzugt 5 bis 20 Gew.-% mikrofeinen Füllstoffen, wie pyrogenen Kieselsäuren mit einer mittleren Korngrösse von 0,02 bis 0,05 µm, sowie 65 bis 89 Gew.-% feinverteiltem anorganischem Füllstoff mit einer mittleren Korngrösse von 1 bis 15 µm, bevorzugt 2 bis 10 µm, ganz besonders bevorzugt 3 bis 8 µm. Die Füllstoffkonzentration bezieht sich auf (a) + (b).

Zum besseren Einbau in die Polymermatrix kann es von Vorteil sein, die Füllstoffe sowie gegebenenfalls röntgenopake Zusatzstoffe zu hydrophobieren. Übliche Hydrophobierungsmittel sind Silane, beispielsweise Trimethoxymethacryloxypropylsilan.

In einer bevorzugten Ausführungsform sind sämtliche eingesetzten anorganischen Füllstoffe silanisiert, vorzugsweise mit Trimethoxymethacryloxypropylsilan. Die Menge des eingesetzten Silans beträgt überlicherweise 0,5 bis 10 Gew.-%, bezogen auf anorganische Füllstoffe, bevorzugt 1 bis 6 %, ganz besonders bevorzugt 2 bis 5 Gew.-%, bezogen auf anorganische Füllstoffe.

Als röntgenopake Zusatzstoffe können insbesondere die in der europäischen Patentveröffentlichungsschrift 238025 genannten Schwermetallfluoride eingesetzt werden.

In einer bevorzugten Ausführungsform der Erfindung werden 5 bis 30 Gew.-% röntgenopake Zusatzstoffe, insbesondere Yttriumfluorid, bezogen auf (a) + (b), eingesetzt, ganz besonders bevorzugt sind 10 bis 25 Gew.-% Yttriumfluorid.

## Beispiel 1 (photopolymerisierbare Zahnfüllmassen)

70 Gewichtsteile Bis-acryloxymethyl-tricyclo-[5.2.1.0.2,6]-decan und 30 Gewichtsteile 2,2-Bis-4-(3-methacryloxy-2-hydroxypropoxy)phenylpropan (Bis-GMA) werden unter vorsichtigem Erwärmen so lange gerührt, bis eine klare Lösung entsteht. Zu der auf Raumtemperatur abgekühlten Lösung werden die in der Tabelle angegebenen Gewichtsprozente an Photoinitiatoren und gegebenenfalls Aktivator gegeben und so lange gerührt, bis eine klare Lösung (1) vorliegt.

20 g dieser Losung (1) werden mit 2 g silanisierter pyrogener Kieselsäure (Aerosil 0 x 50, Fa. Degussa) und 100 g silanisiertem und zahnähnlich pigmentiertem Quarz (mittlere Korngrösse ca. 6µm) zu einer Zahnfüllmasse mit einheitlicher pasteuser Konsistenz verknetet. Tabelle 1 zeigt die physikalischen Messwerte von Pasten, die nach dieser Rezeptur erhalten wurden.

Tabelle 1

| Paste | Photoinitiatoren/ Aktivatoren (Gew.-%, bezogen auf Monomergemisch) | Oberflächenhärte nach DIN 53456 [MPa] | | Druckfestigkeit [MPa] |
|---|---|---|---|---|
| | | 1. Aktivierung x) | 2. Aktivierung xx) | |
| 1 (handelsübliche Masse) | 0,2 Campherchinon<br>1 Dimethylethanolamin | 460 | 475 | 350 |
| 2 (erfindungsgemäss) | 0,2 Campherchinon<br>0,5 Bis-(2,6-dichlorbenzoyl)-4-n-propylphenylphosphinoxid | 123 | 478 | 370 |

x) Belichtung 10 sec. mit ELIPAR®-Gerät (Fa. ESPE), vorgeschaltetem Kantenfilter 475 nm (Durchmesser 8 mm, Dicke 2 mm, Fa. Schott)

xx) Belichtung 20 sec. mit ELIPAR®-Gerät (Fa. ESPE), jedoch ohne vorgeschalteten Kantenfilter

Ergebnis:

Die erfindungsgemässe Paste (2) lässt sich in einem ersten Aktivierungsschritt anhärten, jedoch beträgt die erzielte Härte nach der 1. Aktivierung nur ca. 24 % der erreichbaren Endhärte. Das Material ist nach

diesem Aktivierungsschritt nicht mehr plastisch verformbar, jedoch mit scharfkantigen Instrumenten, wie einem Amalgamschnitzer, noch konturierbar. Es lassen sich feine Fissuren und ein perfekter Rand gestalten. Das Einschleifen der Okklusion mus nicht mit teuren Diamantfinierern, sondern kann auch mit preiswerteren Hartmetallfräsen vorgenommen werden. Nach der optimalen Gestaltung der Oberfläche wird in einem 2. Aktivierungsschritt die vollständige Endhärte erreicht.

Paste (1) (Vergleich) erreicht bereits bei der 1. Aktivierung an der Oberfläche annähernd die Härte, die auch bei der 2. Aktivierung erreicht wird.

Beispiel 2 (röntgenopake photopolymerisierbare Zahnfüllmassen)

9 g von Lösung (1), 6 g silanisierte pyrogene Kieselsäure und 16,5 g fein verteiltes Yttriumfluorid (mittlere Korngrösse ca. 1µm) sowie 60 g silanisierter und zahnähnlich pigmentierter Quarz (mittlere Korngrösse ca. 6 µm ) werden zu einer Zahnfüllmasse mit einheitlich pastöser Konsistenz verknetet. Die physikalischen Messwerte der beiden Pasten mit unterschiedlichen Initiatorkomponenten sind in Tabelle 2 wiedergegeben.

Tabelle 2

| Paste | Photoinitiatoren/ Aktivatoren (Gew.-%, bezogen auf Monomergemisch) | Oberflächenhärte nach DIN 53456 [MPa] | | Druckfestigkeit [MPa] |
|---|---|---|---|---|
| | | 1. Aktivierung x) | 2. Aktivierung xx) | |
| 3 (Vergleich) | 0,2 Campherchinon<br>1 Dimethylethanolamin | 600 | 630 | 400 |
| 4 (erfindungsgemäss) | 0,2 Campherchinon<br>0,5 Bis-(2,6-dichlorbenzoyl)-4-n-propylphenylphosphinoxid | 85 | 630 | 420 |

x) Belichtung 10 sec. mit ELIPAR®-Gerät (Fa. ESPE), vorgeschaltetem Kantenfilter 475 nm (Durchmesser 8 mm, Dicke 2 mm, Fa. Schott)

xx) Belichtung 20 sec. mit ELIPAR®-Gerät (Fa. ESPE), jedoch ohne vorgeschalteten Kantenfilter

Ergebnis:

Die erfindungsgemässe Paste (4) lässt sich in einem ersten Aktivierungschritt anhärten, jedoch beträgt die erzielte Härte nach der 1. Aktivierung nur ca. 14% der erreichbaren Endhärte. Das Material ist nach

diesem Aktivierungsschritt nicht mehr plastisch verformbar, jedoch mit scharfkantigen Instrumenten, wie Amalgamschnitzer, noch konturierbar. Es lassen sich feine Fissuren und ein perfekter Rand gestalten. Das Einschleifen der Okklusion muss nicht mit teuren Diamantfinierern, sondern kann auch mit preiswerteren Hartmetallfräsen vorgenommen werden. Nach der optimalen Gestaltung der Oberfläche wird in einem 2. Aktivierungsschritt die vollständige Endhärte erreicht.

Paste (3) (Vergleich) erreicht bereits bei der 1. Aktivierung an der Oberfläche annähernd die Härte, die auch bei der 2. Aktivierung erreicht wird.

Beispiel 3

50 Gew.-teile Bis-acryloxymethyl-tricyclo-[5.2.1.0.2,6]-decan und 50 Gew.-teile des Umsetzungsproduktes aus 2 Mol Acrylsäure und 1 Mol des bis-ethoxylierten Bis-hydroxymethyl-tricyclo-[5.2.1.0.2,6]-decans werden unter vorsichtigem Erwärmen so lange gerührt, bis eine klare Lösung entsteht. Zu der auf Raumtemperatur abgekühlten Lösung werden 0,6 Gew.-% Campherchinon und 1 Gew.-% Bis-(2,6-dichlorbenzoyl)-4-n-propylphenylphosphinoxid gegeben und solange gerührt, bis eine klare Losung (2) vorliegt.

56 g von Lösung (2), 15 g Calciumfluorid, 9,2 g silanisierte pyrogene Kieselsäure, 30,3 g Yttriumfluorid und 6 g Pigmente werden zu einer Zementierungsmasse mit einheitlicher pastöser, aber fliessfähiger Konsistenz verknetet. Nach einer Belichtung von 20 Sekunden mit dem Elipar ®-Gerät (Fa. ESPE) und vorgeschaltetem Kantenfilter 475 nm (Durchmesser 8 mm, Dicke 2 mm, Fa. Schott) hat das Material eine Oberflächenhärte, gemessen nach DIN 53 456, von 65 MPa und eine angehärtete Schichtdicke von 2,5 mm. In diesem Zustand ist das Material ausgezeichnet schnitzbar, so dass sich Überschüsse vom Zementieren sehr leicht entfernen lassen. Die Filmstärke des Zementierungsmaterials beträgt 10 µm. Nach 20 Sekunden Belichtung mit dem Elipar ®-Gerät (Fa. ESPE), jedoch ohne vorgeschalteten Kantenfilter, beträgt die Endhärte des Materials 100 MPa.

**Patentansprüche**

1. Dentalmassen, enthaltend
   (a) 5 bis 70 Gew.-%, bezogen auf (a) + (b), ethylenisch ungesättigtes, polymerisierbares Monomeres und/oder Polymeres,
   (b) 30 bis 95 Gew.-%, bezogen auf (a) + (b), Füllstoffe,
   (c) gegebenenfalls Pigmente, röntgenopake Zusatzstoffe und/oder Thixotropie-Hilfsmittel,
   (d) 0,05 bis 3 Gew.-%, bezogen auf (a), einer Photoinitiatorkomponente I mit einem Lichtabsorptionsmaximum < 450 nm und einer molaren Extinktion der Lichtabsorption von < 10 bei Wellenlängen von 470 nm und darüber sowie
   (e) 0,05 bis 3 Gew.-%, bezogen auf (a), einer Photoinitiatorkomponente II mit einer molaren Extinktion der Lichtabsorption von > 20 bei mindestens einer Wellenlänge von > 450 nm,
   dadurch gekennzeichnet, daß sie die Photoinitiatorkomponente II nach Art und Menge so ausgewählt enthalten, daß die Photoinitiatorkomponente II zu einem ausgehärteten Material führt, das maximal 70%, vorzugsweise maximal 50 % derjenigen Härte aufweist, die das gleiche Material nach Aushärtung nur mit der jeweils verwendeten Photoinitiatorkomponente I aufweist.

2. Massen nach Anspruch 1, dadurch gekennzeichnet, daß sie als Photoinitiatorkomponente I Bisacylphosphinoxide der allgemeinen Formel

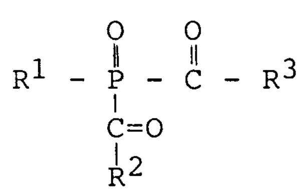

   enthalten, worin bedeuten:

| | |
|---|---|
| $R^1$ einen | geradkettigen oder verzweigten $C_{1-18}$Alkylrest, |
| einen | Cyclohexyl-, Cyclopentyl-, Phenyl-, Naphthyl- oder Biphenylylrest, |
| einen | Cyclopentyl-, Cyclohexyl-, Phenyl-, Naphthyl- oder Biphenylylrest, der substituiert ist durch F, Cl, Br, J, $C_1$-$C_{12}$-Alkyl und/oder $C_1$-$C_{12}$-Alkoxyl, oder |

| | |
|---|---|
| einen | S- oder N-haltigen, 5- oder 6-gliedrigen heterocyclischen Ring und |
| $R^2$ und $R^3$, | die gleich oder verschieden sind, |
| einen | Cyclohexyl-, Cyclopentyl-, Phenyl-, Naphthyl- oder Biphenylylrest, |
| einen | Cyclopentyl-, Cyclohexyl-, Phenyl-, Naphthyl- oder Biphenylylrest, der substituiert ist durch F, Cl, Br, J, $C_{1-4}$-Alkyl und/oder $C_1$-$C_4$-Alkoxyl, oder |
| einen | S- oder N-haltigen, 5- oder 6-gliedrigen heterocyclischen Ring; oder |
| $R^2$ und $R^3$ | miteinander zu einem Ring verknüpft sind, der 4 bis 10 Kohlenstoffatome enthält und durch 1 bis 6 $C_{1-4}$-Alkylreste substituiert sein kann. |

**3.** Massen gemäß Anspruch 2, dadurch gekennzeichnet, daß in der allgemeinen Formel der Bisacylphosphinoxide $R^1$ Decyl, Phenyl, Naphthyl, 4-Biphenylyl, 2-Methylphenyl, 1-Methylnaphthyl, 2,5-Dimethylphenyl, 4-Propylphenyl, 4-Octylphenyl, 4-Chlorphenyl oder 4-Ethoxyphenyl und $R^2$ und $R^3$ Phenyl, Naphthyl, 2,6-Dichlorphenyl, 2,6-Dimethoxyphenyl, 2-Methylnaphthyl, 2-Methoxynaphthyl, 2,6-Dimethylphenyl oder 2,4,6-Trimethylphenyl bedeuten.

**4.** Massen gemäß Anspruch 2 oder 3, dadurch gekennzeichnet, daß in der allgemeinen Formel der Bisacylphosphinoxide $R^2$ und $R^3$ die gleiche Bedeutung aufweisen.

**5.** Massen gemäß Anspruch 1, dadurch gekennzeichnet, daß sie als Photoinitiatorkomponente I Bis(2,6-dichlorbenzoyl)-phenylphosphinoxid, Bis(2,6-dichlorbenzoyl)-2,5-dimethylphenylphosphinoxid und/oder Bis(2,6-dichlorbenzoyl)-4-n-propylphenylphosphinoxid enthalten.

**6.** Massen nach einem der Ansprüche 1 bis 5, dadurch gekennzeichnet, daß sie als Photoinitiatorkomponente II $\alpha$-Diketone, insbesondere Campherchinon, enthalten.

**7.** Massen nach einem der Ansprüche 1 bis 6, dadurch gekennzeichnet, daß sie je 0,1 bis 2, insbesondere 0,5 bis 1,5 Gew.-% der Photoinitiatorkomponenten I und II, bezogen auf (a), enthalten.

**8.** Massen nach einem der Ansprüche 1 bis 7, dadurch gekennzeichnet, daß sie als (c) 5 bis 30 Gew.-%, bezogen auf (a) + (b), röntgenopake Zusatzstoffe enthalten.

**9.** Verfahren zur Herstellung der Dentalmassen gemäß einem der Patentansprüche 1 bis 8, dadurch gekennzeichnet, daß man die Bestandteile (a), (b), (d), (e) und gegebenenfalls (c) miteinander vermischt.

**10.** Verwendung einer Kombination aus einer Photoinitiatorkomponente I mit einem Lichtabsorptionsmaximum < 450 nm und einer molaren Extinktion der Lichtabsorption von < 10 bei Wellenlängen von 470 nm und darüber und aus einer Photoinitiatorkomponente II mit einer molaren Extinktion der Lichtabsorption von > 20 bei mindestens einer Wellenlänge von > 450 nm zur Herstellung von in zwei Schritten härtbaren Dentalmassen gemäß den Ansprüchen 1 bis 8.

**11.** Verfahren zum Härten der Dentalmassen gemäß einem der Ansprüche 1 bis 8, dadurch gekennzeichnet, daß man die Massen in einem ersten Aushärtungsschritt mit Licht aus dem Wellenlängenbereich von im wesentlichen > 450 nm und, nach Oberflächengestaltung der vorgehärteten Dentalmasse, in einem zweiten Aushärtungsschritt mit Licht, das deutliche Anteile von Wellenlängen < 450 nm aufweist, bestrahlt.

## Claims

**1.** Dental compositions containing

(a) 5 to 70 wt.%, relative to (a) + (b), ethylenically unsaturated polymerizable monomer and/or polymer,

(b) 30 to 95 wt.%, relative to (a) + (b), fillers,

(c) optionally pigments, X-ray-opaque additives, and/or thixotropy auxiliaries,

(d) 0.05 to 3 wt.%, relative to (a), of a photoinitiator component I with a light absorption maximum of < 450 nm and a molar extinction of the light absorption of < 10 at wavelengths of 470 nm and above,

(e) 0.05 to 3 wt.%, relative to (a), of a photoinitiator component II with a molar extinction of the light absorption of > 20 at at least a wavelength of > 450 nm,

characterized in that they contain photoinitiator component II selected in a manner and quantity so that photoinitiator component II leads to a fully-cured material which displays at most 70%, preferably at most 50%, of that hardness which the same material displays after curing exclusively with the photoinitiator component I used in each case.

**2.** Compositions according to claim 1, characterized in that they contain as photoinitiator component I bisacylphosphinic oxides of the general formula

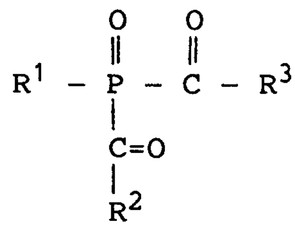

wherein:

$R^1$ is a straight-chained or branched $C_{1-18}$ alkyl group,
a cyclohexyl, cyclopentyl, phenyl, naphthyl or biphenylyl group,
a cyclopentyl, cyclohexyl, phenyl, naphthyl or biphenylyl group which is substituted by F, Cl, Br, I, $C_1$-$C_{12}$ alkyl and/or $C_1$-$C_{12}$ alkoxyl, or
an S- or N-containing, 5- or 6-membered heterocyclic ring, and

$R^2$ and $R^3$, which are the same or different, represent
a cyclohexyl, cyclopentyl, phenyl, naphthyl or biphenylyl group,
a cyclopentyl, cyclohexyl, phenyl, naphthyl or biphenylyl group which is substituted by F, Cl, Br, I, $C_{1-4}$ alkyl and/or $C_1$-$C_4$ alkoxyl, or
an S- or N-containing, 5- or 6- membered heterocyclic ring; or

$R^2$ and $R^3$ are joined together to form a ring which contains 4 to 10 carbon atoms and can be optionally substituted by 1 to 6 $C_{1-4}$ alkyl groups.

**3.** Compositions according to claim 2, characterized in that, in the general formula of the bisacylphosphine oxides, $R^1$ represents decyl, phenyl, naphthyl, 4-biphenylyl, 2-methylphenyl, 1-methylnaphthyl, 2,5-dimethylphenyl, 4-propylphenyl, 4-octylphenyl, 4-chlorophenyl or 4-ethoxyphenyl, and $R^2$ and $R^3$ represent phenyl, naphthyl, 2,6-dichlorophenyl, 2,6-dimethoxyphenyl, 2-methylnaphthyl, 2-methoxynaphthyl, 2,6-dimethylphenyl or 2,4,6-trimethylphenyl.

**4.** Compositions according to claim 2 or 3, characterized in that, in the general formula of the bisacylphosphine oxides, $R^2$ and $R^3$ have the same meaning.

**5.** Compositions according to claim 1, characterized in that they contain, as the photoinitiator component I, bis(2,6-dichlorobenzoyl)-phenylphosphine oxide, bis(2,6-dichlorobenzoyl)-2,5-dimethylphosphine oxide and/or bis(2,6-dichlorobenzoyl)-4-n-propylphenyl-phosphine oxide.

**6.** Compositions according to one of claims 1 to 5, characterized in that they contain α-diketones, in particular camphor quinone, as photoinitiator component II.

**7.** Compositions according to one of claims 1 to 6, characterized in that they contain 0.1 to 2, particularly 0.5 to 1.5 wt.% each, relative to (a), of photoinitiator components I and II.

**8.** Compositions according to one of claims 1 to 7, characterized in that they contain, as (c), 5 to 30 wt.%, relative to (a) + (b), of X-ray-opaque additives.

**9.** Process for producing the dental compositions according to one of patent claims 1 to 8, characterized in that components (a), (b), (d), (e) and optionally (c) are mixed together.

**10.** Use of a combination of a photoinitiator component I with a light absorption maximum of < 450 nm and a molar extinction of the light absorption of < 10 at wavelengths of 470 nm and above, and a photoinitiator component II with a molar extinction of the light absorption of > 20 at at least a wavelength of > 450 nm to produce dental compositions, curable in two steps according to claims 1 to 8.

**11.** Process for curing the dental compositions according to one of claims 1 to 8, characterized in that the compositions are irradiated in a first curing step with light from the wavelength range of substantially > 450 nm and, after the surface of the pre-cured dental composition has formed, in a second curing step with light which displays clear portions of wavelengths of < 450 nm.

**Revendications**

**1.** Masses dentaires contenant :
(a) 5 à 70 % en poids, par rapport à (a) + (b), d'un monomère polymérisable et/ou d'un polymère éthyléniquement insaturé,
(b) 30 à 95 % en poids, par rapport à (a) + (b), de charges,
(c) éventuellement des pigments, des additifs opaques aux rayons X et/ou un auxiliaire de thixotropie,
(d) 0,05 à 3 % en poids, par rapport à (a), d'un composant photo-initiateur I ayant une valeur maximale d'absorption de lumière < 450 nm et un coefficient d'extinction moléculaire de l'absorption de lumière < 10 à des longueurs d'onde de 470 nm et au-delà, ainsi que
(e) 0,05 à 3 % en poids, par rapport à (a), d'un composant photo-initiateur II ayant un coefficient d'extinction moléculaire de l'absorption de lumière > 20, au moins à une longueur d'onde > 450 nm,
caractérisées en ce qu'elles contiennent le composant photo-initiateur II dont la nature et la quantité sont choisies de manière telle que le composant photo-initiateur II conduit à un matériau durci dont la dureté vaut 70 % au plus, de préférence 50 % au plus, de la dureté du même matériau après son durcissement seulement au moyen du composant photo-initiateur I utilisé à chaque fois.

**2.** Masses selon la revendication 1, caractérisées en ce qu'elles contiennent, comme composant photo-initiateur I, des oxydes de bisacylphosphine répondant à la formule générale :

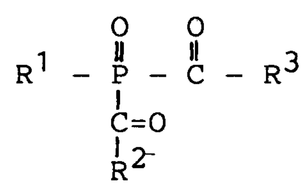

dans laquelle :

$R^1$ signifie un reste alkyle en $C_{1-18}$ à chaîne droite ou ramifiée, un reste cyclohexyle, cyclopentyle, phényle, naphtyle ou biphénylyle,
un reste cyclopentyle, cyclohexyle, phényle, naphtyle ou biphénylyle qui est substitué par F, Cl, Br, J, alkyle en $C_1$-$C_{12}$ et/ou alcoxyle en $C_1$-$C_{12}$, ou
un composé hétérocyclique à 5 ou 6 chaînons et contenant S ou N ; et

$R^2$ et $R^3$, qui sont identiques ou différents, signifient un reste cyclohexyle, cyclopentyle, phényle, naphtyle ou biphénylyle,
un reste cyclopentyle, cyclohexyle, phényle, napthyle ou biphénylyle qui est substitué par F, Cl, Br, J, alkyle en $C_1$-$C_4$ et/ou alcoxyle en $C_1$-$C_4$, ou
un composé hétérocyclique à 5 ou 6 chaînons et contenant S ou N ; ou

$R^2$ et $R^3$ sont condensés en un composé cyclique qui contient de 4 à 10 atomes de carbone et peut être substitué par 1 à 6 restes alkyles en $C_1$-$C_4$.

**3.** Masses selon la revendication 2, caractérisées en ce que, dans la formule générale des oxydes de bisacylphosphine, $R^1$ signifie un reste décyle, phényle, naphtyle, 4-biphénylyle, 2-méthylphényle, 1-méthylnaphtyle, 2,5-diméthylphényle, 4-propylphényle, 4-octylphényle, 4-chlorophényle ou 4-éthoxyphényle, et $R^2$ et $R^3$ signifient un reste phényle, naphtyle, 2,6-dichlorophényle, 2,6-diméthoxyphényle,

2-méthylnaphtyle, 2-méthoxynaphtyle, 2,6-diméthylphényle ou 2,4,6-triméthylphényle.

4. Masses selon la revendication 2 ou 3, caractérisées en ce que, dans la formule générale des oxydes de bisacylphosphine, $R^2$ et $R^3$ ont la même signification.

5. Masses selon la revendication 1, caractérisées en ce qu'elles contiennent, comme composant photo-initiateur I, l'oxyde de bis(2,6-dichlorobenzoyl)phénylphosphine, l'oxyde de bis(2,6-dichlorobenzoyl)-2,5-diméthylphénylphosphine et/ou l'oxyde de bis(2,6-dichlorobenzoyl)-4-n-propylphénylphosphine.

6. Masses selon une des revendications 1 à 5, caractérisées en ce qu'elles contiennent, comme composant photo-initiateur II, des $\alpha$-dicétones, en particulier la camphoquinone.

7. Masses selon une des revendications 1 à 6, caractérisées en ce qu'elles contiennent de 0,1 à 2, en particulier de 0,5 à 1,5, % en poids respectivement des composants photo-initiateurs I et II, par rapport à (a).

8. Masses selon une des revendications 1 à 7, caractérisées en ce qu'elles contiennent, comme composant (c), de 5 à 30 % en poids, par rapport à (a) + (b), d'additifs opaques aux rayons X.

9. Procédé de préparation des masses dentaires selon une des revendications 1 à 8, caractérisé en ce qu'on mélange ensemble les constituants (a), (b), (d), (e) et éventuellement (c).

10. Utilisation d'une combinaison d'un composant photo-initiateur I ayant une valeur maximale d'absorption de lumière < 450 nm et un coefficient d'extinction moléculaire de l'absorption de lumière < 10 à des longueurs d'onde de 470 nm et au-delà, et d'un composant photo-initiateur II ayant un coefficient d'extinction moléculaire de la lumière d'absorption > 20, au moins à une longueur d'onde > 450 nm, pour la préparation de masses dentaires durcissables en deux étapes selon les revendications 1 à 8.

11. Procédé de durcissement des masses dentaires selon une des revendications 1 à 8, caractérisé en ce qu'on irradie la masse, lors d'une première étape de durcissement, avec une lumière comprise dans la plage de longueurs d'onde sensiblement > 450 nm et, après le façonnage superficiel de la masse dentaire prédurcie, lors d'une deuxième étape de durcissement, avec une lumière qui comporte des fractions nettes de longueurs d'onde < 450 nm.